# EUROPÄISCHE PATENTANMELDUNG

(11) **EP 4 686 753 A1**
(43) Veröffentlichungstag der Anmeldung: **04.02.2026**
(21) Anmeldenummer: 25190361.3
(22) Anmeldetag: 18.07.2025
(51) Int. Cl.: C12M 1/00, C12M 1/42

(54) **BIOREAKTORSYSTEM, PROBENKAMMER HIERFÜR UND VERWENDUNG EINER SOLCHEN PROBENKAMMER**

(30) Priorität: 02.08.2024 DE 102024207351
(71) Anmelder: Friedrich-Alexander-Universität Erlangen-Nürnberg, in Vertretung des Freistaates Bayern, 91058 Erlangen (DE)
(72) Erfinder: BAUER, Julian, 91058 Erlangen (DE); FRIEDRICH, Oliver, 91058 Erlangen (DE); LESKO, Christian, 91058 Erlangen (DE); RITTER, Paul, 91058 Erlangen (DE); SCHNEIDEREIT, Dominik, 91058 Erlangen (DE); SCHUBERT, Dirk Wolfram, 91058 Erlangen (DE); WERNER, Siegfried, 91058 Erlangen (DE); HOLZNER, Andreas, 91058 Erlangen (DE)
(74) Vertreter: FDST Patentanwälte

(57) **Zusammenfassung**

Es wird ein Bioreaktorsystem (2) angegeben, welches eine Probenkammer (4) aufweist, mit einem Innenraum (6) zum Aufnehmen einer Probe (8), welches eine Halterung (12) aufweist, in welche die Probenkammer (4) eingesetzt ist, welches eine Untersuchungseinheit (14) aufweist, zur Untersuchung der Probe (8), wobei die Probenkammer (4) wenigstens eine Kammerwand (18) aufweist, welche elastisch ist, um beim Verfahren der Untersuchungseinheit (14) entlang eines ersten Verfahrwegs (V1) durch die Untersuchungseinheit (14) verformt zu werden. Weiter werden eine Probenkammer (4) und eine Verwendung für eine solche Probenkammer (4) angegeben.

## Beschreibung

Die Erfindung betrifft ein Bioreaktorsystem, beispielsweise zur Bioanalytik von Biofabrikaten, z.B. Gewebekonstrukten. Weiter betrifft die Erfindung eine Probenkammer für solch ein Bioreaktorsystem sowie eine spezielle Verwendung der Probenkammer.

Ein Bioreaktorsystem, kurz Bioreaktor, dient zur Kultivierung und Untersuchung von biologischen Proben, z.B. Gewebekonstrukten. Hierfür weist das Bioreaktorsystem eine Probenkammer auf, in welche die Probe zur Untersuchung aufgenommen ist. Die Probenkammer stellt eine geeignete Umgebung für die Probe bereit, in welcher die Probe dann untersucht wird. Die Untersuchung erfolgt typischerweise mittels einer Untersuchungseinheit des Bioreaktorsystems.

Bioreaktorsysteme mit Zellkulturkammern mit optischem Zugang weisen typischerweise ein Deckglas auf, auf welches ein Polymer-Rahmen (z.B. Polystyrol, Polymethylacrylat, Polycarbonat) mit einer Vielzahl an Zellkulturkammern aufgesetzt wird. Dies ermöglicht eine Inkubation von Zellen oder Geweben mit anschließender optischer Analyse mit einem inversen Mikroskopie-System. Diese modularen Mikroskopiekammern können auch in Verbindung mit halbautomatisierten optischen Systemen verwendet werden, welche simultane Mikroskopie und Inkubation ermöglichen. Doch selbst in Verbindung mit einer Mikroskopieumgebung weisen solche Mehrkammer-Objektträgersysteme die üblichen Nachteile herkömmlicher Zellkultur auf. Der Wechsel des Kulturmediums erfolgt chargenweise manuell als batch-Prozess, auftretende Temperaturschwankungen durch das Öffnen und Schließen des Inkubators während dem Wechsel des Mediums beeinflussen die Homöostase der biologischen Proben, die optische Auflösung ist begrenzt durch die erforderlichen Arbeitsabstände der Objektive und damit niedriger numerischer Aperturen, und die anwendbaren Bildgebungsmodalitäten sind bei kleiner Kammergeometrie in Mehrkammer-Systemen auf inverse optische Systeme eingeschränkt. All diese Einflussfaktoren führen im Zusammenspiel zu einem inhomogenen Inkubationsprozess mit unvorhersehbaren Auswirkungen auf die Gewebereifung oder -funktionalität.

Es wird verwiesen auf:
KENSAH, G., GRUH, 1., VIERING, J., SCHUMANN, H., DAHLMANN, J., MEYER, H., SKVORC, D., BÄR, A., AKHYARI, P., HEISTERKAMP, A., AXEL, H. & MARTIN, U. 2011. A Novel Miniaturized Multimodal Bioreactor for Continuous In Situ Assessment of Bioartificial Cardiac Tissue During Stimulation and Maturation. Tissue Engineering Part C: Methods, 17, 463-473.
LAGANA, M. & RAIMONDI, M. T. 2012. A miniaturized, optically accessible bioreactor for systematic 3D tissue engineering research. Biomedical Microdevices, 14, 225-234.
PATEN, J. A., ZAREIAN, R., SAEIDI, N., MELOTTI, S. A. & RUBERTI, J. W. 2011. Design and Performance of an Optically Accessible, Low-Volume, Mechanobioreactor for Lang-Term Study of Living Constructs. Tissue Engineering Part C: Methods, 17, 775-788.
POWERS, M. J., DOMANSKY, K., KAAZEMPUR-MOFRAD, M. R., KALEZI, A., CAPITANO, A., UPADHYAYA, A., KURZAWSKI, P., WACK, K. E., STOLZ, D. B., KAMM, R. & GRIFFITH, L. G. 2002. A microfabricated array bioreactor for perfused 3D liver culture. Biotechnology and Bioengineering, 78, 257-269.
SCHUERLEIN, S., SCHWARZ, T., KRZIMINSKI, S., GÄTZNER, S., HOPPENSACK, A., SCHWEDHELM, I., SCHWEINLIN, M., WALLES, H. & HANSMANN, J. 2017. A versatile modular bioreactor platform for Tissue Engineering. Biotechnology Journal, 12, 1600326.
STEPHENS, J. S., COOPER, J. A., PH ELAN JR., F. R. & DUNKERS, J. P. 2007. Perfusion flow bioreactor for 3D in situ imaging: Investigating cell/biomaterials interactions. Biotechnology and Bioengineering, 97, 952-961.

Bioreaktorsysteme zur Kultivierung von Biofabrikaten sind somit grundsätzlich bekannt und unterscheiden sich vorwiegend hinsichtlich des spezifischen Anwendungsbereichs sowie des Reaktorvolumens. Auch sind optisch zugängliche Mikrobioreaktorkammern grundsätzlich verfügbar. Diese sind allerdings in ihrer Anwendung auf spezielle Fälle beschränkt.

Weiterhin wird verwiesen auf PT 117857 A, US 2020 / 354 660 A1, US 2021 / 369 917 A1, WO 2019 / 237 061 A1, DE 20 2018 001 671 U1, US 2018 / 216 057 A1, PT 106827 A, US 2012 / 100 602 A1, US 2010 / 129 899 A1, US 2009 / 215 104 A1.

Vor diesem Hintergrund ist es eine Aufgabe der Erfindung, ein verbessertes Bioreaktorsystem anzugeben, welches insbesondere einen oder mehrere der eingangs genannten Nachteile adressiert. Speziell soll eine Probe innerhalb einer Probenkammer des Bioreaktorsystems möglichst gut für eine Untersuchung zugänglich sein. Hierzu sollen ein entsprechend verbessertes Bioreaktorsystem sowie eine Probenkammer hierfür angegeben werden. Weiter soll eine spezielle Verwendung der Probenkammer angegeben werden.

Die Aufgabe wird erfindungsgemäß gelöst durch ein Bioreaktorsystem mit den Merkmalen gemäß Anspruch 1, durch eine Probenkammer mit den Merkmalen gemäß Anspruch 14 sowie durch eine Verwendung mit den Merkmalen gemäß Anspruch 15. Vorteilhafte Ausgestaltungen, Weiterbildungen und Varianten sind Gegenstand der Unteransprüche. Die Ausführungen im Zusammenhang mit dem Bioreaktorsystem gelten sinngemäß auch für die Probenkammer und die Verwendung sowie umgekehrt.

Ein erfindungsgemäßes Bioreaktorsystem weist eine Probenkammer auf, mit einem Innenraum zum Aufnehmen einer Probe. Die Probenkammer weist hierfür eine Wandung auf, welche den Innenraum umschließt und dessen Form definiert. Die Probe ist vorzugsweise ein insbesondere dreidimensionales Biofabrikat und/oder Gewebekonstrukt. Das Bioreaktorsystem wird kurz auch lediglich als "Bioreaktor" bezeichnet, die Probenkammer wird auch als "Reaktorkammer" oder "Bioreaktionskammer" bezeichnet. Die Probenkammer ist insbesondere abgeschlossen.

Weiter weist das Bioreaktorsystem eine Halterung auf, in welche die Probenkammer eingesetzt ist, vorzugsweise formschlüssig und/oder dicht. Im eingesetzten Zustand ist die Probenkammer in der Halterung fixiert. Schließlich weist das Bioreaktorsystem noch eine Untersuchungseinheit auf, zur Untersuchung der Probe, während diese in der Probenkammer aufgenommen ist, d.h. platziert ist. Mit der Untersuchungseinheit wird die Probe im Betrieb untersucht, vorzugsweise im laufenden Betrieb und/oder kontinuierlich im Betrieb. Die Untersuchungseinheit ist insbesondere ein Teil einer Messvorrichtung des Bioreaktorsystems, mit welchem die Probe analysiert wird, d.h. mit welchem eine oder mehrere Eigenschaften der Probe ermittelt werden.

In die Halterung sind geeigneterweise noch ein oder mehrere Sensoren und/oder Aktoren integriert, um je nach Bedarf die Probe und/oder deren Umgebung (z.B. ein Medium im Innenraum) zu untersuchen, zu stimulieren und/oder zu manipulieren. Ein Beispiel ist ein Temperatursensor zur Messung der Temperatur der Probenkammer. Ein weiteres Beispiel ist ein Heizelement, z.B. Peltierelement, zum Beheizen der Probenkammer und somit auch des Innenraums und der Probe. Jegliche Sensoren und Aktoren sind zweckmäßigerweise vollständig außerhalb der Probenkammer angeordnet, dringen also nicht in den Innenraum ein, was aber als Alternative gleichwohl auch möglich ist.

Die Probenkammer weist wenigstens eine (erste) Kammerwand auf, welche ganz oder teilweise elastisch (insbesondere flexibel) ist, um beim Verfahren der Untersuchungseinheit entlang eines ersten Verfahrwegs durch die Untersuchungseinheit verformt zu werden. Diese erste Kammerwand wird auch als "elastische Kammerwand" bezeichnet. Die Kammerwand ist ein Teil der Wandung der Probenkammer. In einem unverformten Zustand ist die Kammerwand insbesondere plan, d.h. flach oder eben. Insbesondere weist die Probenkammer mehrere Kammerwände auf, welche zusammen die Wandung bilden und den Innenraum umschließen.

Mehrere oder sämtliche Kammerwände der Probenkammern können grundsätzlich gleichartig ausgebildet sein.

In einer bevorzugten Ausgestaltung ist die Probenkammer insgesamt quaderförmig, mit dann sechs Kammerwänden, von welchen dann wenigstens eine elastisch ist. Andere Formen für die Probenkammer und andere Anzahlen an Kammerwänden sind aber denkbar und auch grundsätzlich geeignet. Eine quaderförmige Ausgestaltung ist jedoch vorteilhaft, da eine solche besonders einfach zu handhaben ist, speziell ist die Halterung des Bioreaktorsystems dann besonders einfach gestaltbar, z.B. ebenfalls quaderförmig. Außerdem ist eine quaderförmige Probenkammer besonders kompakt und die insbesondere flächigen, ebenen Kammerwände ermöglichen eine verzerrungsfreie Untersuchung, insbesondere optische Untersuchung. Eine Quaderform ist abseits der Untersuchung aber auch während der Präparierung und Kultivierung der Probe vorteilhaft.

Ein besonderer Vorteil der elastischen Kammerwand ist insbesondere, dass die Untersuchungseinheit nun deutlich näher an die Probe herangefahren werden kann und auch ein größerer Verfahrweg für die Untersuchungseinheit möglich ist als im Vergleich zu einer nicht-elastischen und somit nicht-verformbaren Kammerwand. Vorzugsweise ist die Untersuchungseinheit ausgebildet zur optischen Untersuchung der Probe. Besonders geeignet ist hierfür eine Untersuchungseinheit, welche ein Objektiv ist, insbesondere Mikroskop-Objektiv, wovon nachfolgend ohne Beschränkung der Allgemeinheit auch ausgegangen wird. Die Ausführungen im Zusammenhang mit einem Objektiv gelten auch analog für jegliche Untersuchungseinheit, welche zur optischen Untersuchung ausgebildet ist. Alternativ ist als Untersuchungseinheit auch ein Spektroskop geeignet, mit welchem ebenfalls eine optische Untersuchung der Probe möglich ist. Gleichwohl gelten die Ausführungen und Vorteile auch für andere Untersuchungseinheiten, z.B. einen Sensor (optisch, elektrisch, magnetisch usw.), einen Fühler, eine Glasfaser oder ähnliches. Die Probe ist innerhalb der Probenkammer insbesondere nicht zwingend unmittelbar an der elastischen Kammerwand positioniert, sondern zweckmäßigerweise in einem Abstand davon, z.B. im Zentrum der Probenkammer oder an einer der elastischen Kammerwand gegenüberliegenden Kammerwand. Dieser Abstand wird nun durch das Anfahren der elastischen Kammerwand und deren Verformung durch die Untersuchungseinheit vorteilhaft reduziert, sodass eine verbesserte Untersuchung der Probe möglich ist.

Die Wandung der Probenkammer ist vorzugsweise dünnwandig, d.h. dünner als breit und/oder hoch, z.B. liegt eine Dicke der Wandung im Bereich von 0,2 mm bis 1 mm. Die Probenkammer, genauer deren Innenraum, weist bevorzugterweise ein Volumen im Bereich von 1 ml bis 10 ml auf, andere Volumina sind aber grundsätzlich auch möglich. Für die Probenkammer ergeben sich dann vorteilhafte Abmessungen (Höhe, Breite, Länge) im Bereich von 0,1 mm bis zu 50 mm, vorzugsweise wenigsten 10 mm, andere Abmessungen sind aber grundsätzlich auch möglich. Relativ zueinander unterscheiden sich die Abmessungen der Probenkammer zweckmäßigerweise maximal um einen Faktor 2, sodass die Probenkammer insgesamt näherungsweise würfelförmig erscheint und jedenfalls nicht flach ist, sondern eine dreidimensionale Probe aufnehmen kann.

Die genannten Abmessungen bis in den zweistelligen Millimeterbereich (≥ 10 mm) hinein, stellen eine besondere Herausforderung für die optische Untersuchung, insbesondere Bildgebung dar, da hochauflösende Objektive in der Regel über Arbeitsabstände im einstelligen Millimeterbereich (< 10 mm) verfügen. Um diese Herausforderung zu umgehen, sind vorliegend eine oder mehrere der Kammerwände elastisch, d.h. aus einem elastischen Material gefertigt, welches eine elastische Verformung durch das Objektiv erlaubt. Das Objektiv kann beim Anfahren insbesondere eine oder mehrere Kammerwände durch direkten Kontakt insbesondere der Objektivlinse des Objektivs elastisch verformen und somit den Abstand zwischen Objektiv und Probe unabhängig von den Abmessungen der Probenkammer variieren. Diese Möglichkeit erlaubt es, auch bei im Vergleich zum Arbeitsabstand relativ großem Volumen des Innenraums (d.h. Abmessungen der Probenkammer) noch hochauflösende Objektive mit dann entsprechend geringem Arbeitsabstand zu verwenden.

In unverformtem Zustand der Probenkammer liegt die elastische Kammerwand insbesondere entlang des ersten Verfahrwegs. Beim Anfahren der elastischen Kammerwand mit der Untersuchungseinheit stößt diese an die Kammerwand an und drückt entlang des Verfahrwegs auf die Kammerwand, sodass diese entsprechend verformt wird. Der Verfahrweg weist demnach einen Kontaktpunkt auf, welcher den Verfahrweg in zwei aufeinanderfolgende Abschnitte teilt, nämlich einen ersten Abschnitt, entlang welchem die Untersuchungseinheit ohne jeglichen Kontakt zur Kammerwand verfahren wird, und einen zweiten Abschnitt, entlang welchem die Untersuchungseinheit an der Kammerwand anliegt und diese beim weiteren Verfahren entsprechend verformt. Dabei liegt die Untersuchungseinheit an der Kammerwand auf einer Kontaktfläche an, welche geringer ist als eine Gesamtfläche der Kammerwand. Die Kontaktfläche beträgt z.B. maximal 50 % der Gesamtfläche.

Die Kammerwand wird beim Anfahren insbesondere in den Innenraum hinein und insbesondere auf die Probe zu verformt. Die elastische Kammerwand ist insbesondere reversibel verformbar. Beim Zurückfahren der Untersuchungseinheit entlang des Verfahrwegs nimmt die Kammerwand demnach einen ursprünglichen, unverformten Zustand wieder an. Die elastische Kammerwand ist insbesondere auch zerstörungsfrei verformbar. Die Kammerwand wird demnach nicht durch die Untersuchungseinheit beschädigt, z.B. punktiert, zerteilt oder dergleichen, sondern bleibt intakt und geschlossen, sodass insbesondere auch der Innenraum der Probenkammer insgesamt geschlossen bleibt.

Grundsätzlich ist es vorteilhaft, wenn auch noch weitere oder sogar alle Kammerwände der Probenkammer analog zur beschriebenen elastischen Kammerwand ebenfalls jeweils elastisch sind. Die Untersuchungseinheit oder eine andere Einheit kann dann analog diese anderen elastischen Kammerwände anfahren und dabei entsprechend verformen.

Da die Probenkammer wenigstens während der Untersuchung regelmäßig vollständig mit einem typischerweise flüssigen und daher inkompressiblen Medium gefüllt ist, ist es vorteilhaft, wenn auch wenigstens eine weitere Kammerwand elastisch ist, sodass für das Medium eine entsprechende Ausweichmöglichkeit vorhanden ist.

Die Untersuchungseinheit ist zweckmäßigerweise außerhalb der Probenkammer angeordnet, wodurch vermieden wird, dass einerseits die Untersuchungseinheit die Probe und deren Umgebung kontaminiert und dass andererseits die Untersuchungseinheit durch die Probe oder das Medium verschmutzt wird. Ein unmittelbarer Kontakt der Untersuchungseinheit mit der Probe oder mit dem Medium, welches die Probe umgibt, wird also vermieden.

In einer besonders einfachen Ausgestaltung verläuft der erste Verfahrweg senkrecht zur Kammerwand und ist damit auch besonders kurz. Zugleich werden zusätzliche Scherkräfte auf die Kammerwand beim Anfahren vermieden.

Die Untersuchungseinheit weist allgemein insbesondere einen Arbeitsabstand auf, d.h. einen Abstand, welcher zwischen der Untersuchungseinheit und der Probe einzustellen ist, damit die Untersuchungseinheit verwendet werden kann. Im Falle eines Objektivs ist der Arbeitsabstand dessen Brennweite oder zumindest davon abgeleitet. Der Arbeitsabstand ist vorzugsweise geringer als ein Abstand zwischen der Kammerwand in unverformtem Zustand und einer Probenposition für die Probe innerhalb der Probenkammer, wobei der Abstand insbesondere entlang einer Achse von der Probe zur Untersuchungseinheit gemessen ist. Mit anderen Worten: der Arbeitsabstand ist geringer als der in unverformtem Zustand hin zur Probe zu überbrückende Abstand innerhalb des Innenraums. Dieser zu überbrückende Abstand wird nun beim Anfahren der Kammerwand durch die elastisch ausgebildete Kammerwand reduziert, sodass die Untersuchungseinheit nutzbar ist. Aufgrund der elastischen Kammerwand ist also eine größere Auswahl an Untersuchungseinheiten, speziell an Arbeitsabständen, zugänglich. Im Falle eines Objektivs können demnach vorteilhaft unabhängig von den Abmessungen der Probenkammer in unverformtem Zustand im Grunde Objektive mit verschiedenen Brennweiten genutzt werden, d.h. allgemein Untersuchungseinheiten mit beliebigem Arbeitsabstand. Die Abmessungen der Probenkammer und die Positionierung der Probe im Innenraum der Probenkammer beschränken die Auswahl der Untersuchungseinheit und speziell des Arbeitsabstands somit nicht.

Durch die elastische Kammerwand wird demnach vorteilhaft eine optische Untersuchung mittels eines hochauflösenden Objektivs ermöglicht, welches einen nur geringen Arbeitsabstand aufweist. In der Regel korreliert der Arbeitsabstand eines Objektivs direkt mit der numerischen Apertur, je höher die numerische Apertur und somit auch die optische Auflösung ist, desto geringer ist der Arbeitsabstand. Das hat zur Folge, dass bisher für die Betrachtung von voluminösen und/oder in ein Medium eingetauchten Proben sogar Objektive mit geringer numerischer Apertur und langen Arbeitsabständen genutzt werden müssen, um die Distanz zwischen Flüssigkeits- und Probenoberfläche zu überbrücken. Die elastische Kammerwand erlaubt nun eine elastische Verformung durch direkten Kontakt mit dem Objektiv, somit kann die Distanz zwischen den Oberflächen (Abstand zwischen Untersuchungseinheit und Probe) direkt verringert werden, und hochauflösende Objektive mit geringen Arbeitsabständen sind einsetzbar. Durch die optionale Einbringung einer Elektrodenanordnung in die Kammerwände, ist auch eine Untersuchung, speziell Bildgebung während aktiver Stimulation der Probe möglich.

Die elastische Kammerwand ist dadurch elastisch, dass diese aus einem elastischen Material hergestellt ist. Ein besonders geeignetes Material ist Polydimethylsiloxan (PDMS) oder Liquid Silicone Rubber (kurz: LSR, ein flexibles Silikon), d.h. wenigstens die elastische Kammerwand ist aus Polydimethylsiloxan oder Liquid Silicone Rubber hergestellt. Ein geeignetes LSR ist insbesondere ein Silikon, welches auf Polydimethylsiloxan (PDMS) basiert und welches auch medizintechnisch zugelassen ist. Dabei bildet PDMS das chemischen Grundgerüst des LSR. LSR ist insbesondere elastisch und zugleich auch vorteilhaft lichtdurchlässig, transparent und biokompatibel. LSR lässt sich außerdem gut in einem Spritzgussverfahren zur Herstellung der Probenkammer verarbeiten. Alternativ zu LSR ist auch PDMS an sich als Material für die Kammerwand geeignet sowie auch jedes zu LSR oder PDMS vergleichbare Silikon oder silikonhaltige Material. Ganz allgemein ist das Material demnach ein silikonhaltiges Material, wobei dieser Begriff auch reine Silikone umfasst.

Geeigneterweise ist die elastische Kammerwand lichtdurchlässig, d.h. transparent insbesondere für sichtbares Licht, IR-Strahlung und/oder UV-Strahlung. Auf diese Weise lässt sich die Probe durch die Kammerwand hindurch optisch untersuchen.

Wie bereits oben angedeutet ist die Untersuchungseinheit bevorzugterweise zur optischen Untersuchung der Probe ausgebildet und insbesondere ein Objektiv. Ein Objektiv dient generell zur optischen Untersuchung. Das Objektiv ist vorzugsweise ein Teil der Messvorrichtung des Bioreaktorsystems. Diese Messvorrichtung ist dann insbesondere ein Mikroskop. Grundsätzlich ist jedes beliebige Mikroskop in dem Bioreaktorsystem nutzbar, z.B. sowohl aufrechte als auch inverse Mikroskop-Systeme oder auch ein Lichtblattmikroskop.

In einer besonders vorteilhaften Ausgestaltung weist die Probenkammer zusätzlich zu der elastischen Kammerwand wenigstens eine weitere (zweite) Kammerwand auf, welche winklig, insbesondere senkrecht, zur elastischen Kammerwand angeordnet ist und lichtdurchlässig ist, zur optischen Untersuchung der Probe (Lichtblatt). Bei der optischen Untersuchung wird die Probe dann insbesondere durch diese weitere Kammerwand beleuchtet, vorzugsweise mit einem sogenannten Lichtblatt. In Verbindung mit einem Objektiv als Untersuchungseinheit wird dann vorteilhafterweise eine Lichtblattmikroskopie (light sheet microscopy) durchgeführt.

Vorzugsweise bestehen jegliche Teile der Probenkammer, welche an den Innenraum angrenzen, d.h. welche eine Innenseite der Probenkammer bilden und somit den Innenraum definieren, aus demselben Material. Dieses Material ist geeigneterweise - wie bereits erwähnt - ein elastisches, lichtdurchlässiges und/oder biokompatibles Material, insbesondere Polydimethylsiloxan oder LSR. Damit ist eine optimale Umgebung für die Probe geschaffen, welche dabei auch aus verschiedenen Richtungen untersucht werden kann. Zugleich ist eine solche Probenkammer besonders einfach zu fertigen, da im Grunde nur ein einziges Material benötigt wird. Andere Teile der Probenkammer, welche nicht an den Innenraum angrenzen, sondern entsprechend außenseitig liegen, speziell die unten noch näher beschriebene Haltekontur, können hingegen aus einem anderen Material bestehen.

Eine solche Probenkammer wird dann geeigneterweise in einem Mehrkomponenten-Spritzgussverfahren, z.B. einem 2K-Spritzgussverfahren hergestellt. Bevorzugterweise ist auch die Anlagekontur oder das gesamte Unterteil aus dem beschriebenen elastischen, lichtdurchlässigen und/oder biokompatiblen Material hergestellt.

Vorzugsweise ist die Probenkammer austauschbar, d.h. die Probenkammer ist lösbar in der Halterung fixiert und kann bei Bedarf aus der Halterung entnommen werden, um dann eine andere, insbesondere aber gleichartige Probenkammer in diese Halterung einzusetzen und entsprechend eine andere Probe zu untersuchen. Auch ist auf diese Weise ein Einwegsystem (single-use-System) realisierbar, bei welchem die Probenkammer immer nur für eine einzige Probe verwendet wird und dann entsorgt wird. Für die nächste Probe wird dann eine neue Probenkammer verwendet.

Zweckmäßigerweise weist die Probenkammer eine Haltekontur, insbesondere einen Rahmen, aus einem starren Material auf, zur (lösbaren) Fixierung der Probenkammer an einem oder mehreren Fixierelementen der Halterung. Das starre Material ist z.B. PP (Polypropylen) oder ein anderer inelastischer Kunststoff. Das starre Material ist wenigstens im Vergleich zum elastischen Material inelastisch und weist hierzu insbesondere einen E-Modul im Bereich von 300 MPa und 1500 MPa auf. Das starre Material ist vorzugsweise derart ausgewählt, dass dessen Dauergebrauchstemperatur deutlich über Raumtemperatur liegt und/ oder dass dieses dielektrische Eigenschaften aufweist, welche bei Anwendung von externen Feldern (statisch oder dynamisch, elektrisch und/oder magnetisch) keine signifikante Feldverzerrung oder Absorption zur Folge hat. Geeignet sind insbesondere das bereits genannte PP sowie PE (Polyethylen), PS (Polystyrol), Harz auf Methacrylatbasis oder Vergleichbare.

Der Rahmen ist zur Ausbildung einer der Kammerwände insbesondere mit einem Material ausgefüllt, vorzugsweise das bereits beschriebene Material für die elastische Kammerwand. Alternativ oder zusätzlich ist in den Rahmen ein Deckglas eingesetzt, z.B. eingeklebt. Insgesamt ist bildet der Rahmen mit dem darin angeordneten Material ein Fenster.

Die Haltekontur und die Fixierelemente bilden geeigneterweise einen Fixiermechanismus, z.B. einen Rastmechanismus. **In** einer geeigneten Ausgestaltung weisen die Fixierelemente jeweils einen Haltefortsatz auf und sind hierzu z.B. als Schnapphaken oder als Rasthaken ausgebildet. Die Haltefortsätze hintergreifen die Haltekontur und fixieren auf diese Weise die Probenkammer in der Halterung und halten somit die Probenkammer in einer bestimmten Position fest. Die Probenkammer wird dann in einer Einsetzrichtung in die Halterung eingesetzt, dabei drückt die Haltekontur entsprechende Haltefortsätze der Fixierelemente zur Seite und gleitet an diesen vorbei. Sobald die Haltekontur die Haltefortsätze passiert hat, rasten die Fixierelemente an der Haltekontur ein und fixieren die Probenkammer, insbesondere in Verbindung mit einem Grund der Halterung, welcher als Gegenkontur zur Fixierung dient. Durch entsprechendes Aufbiegen der Fixierelemente wird die Probenkammer dann wieder gelöst und kann entgegen der Einsetzrichtung wieder entnommen werden.

Vorzugsweise weist die Halterung einen Medienanschluss auf, zur Zufuhr und/oder Abfuhr eines Mediums, d.h. zum Medienmanagement innerhalb der Probenkammer. Der Medienanschluss ist insbesondere ein Teil eines Fluidiksystems des Bioreaktorsystems. Das Medium wird mittels des Medienanschlusses z.B. bereitgestellt und/oder umgewälzt. Insbesondere weist der Medienanschluss einen Vorlauf auf, über welchen das Medium in den Innenraum geführt wird, und einen Rücklauf, über welchen das Medium aus dem Innenraum herausgeführt wird. Das Medium ist z.B. ein Zellkulturmedium oder ein anderes, insbesondere niedrigviskoses Medium. Das Medium ist zweckmäßigerweise in einem Medienreservoir des Bioreaktorsystems gelagert, welches dann mit dem Medienanschluss verbunden ist. Zum Befüllen oder Entleeren des Medienreservoirs weist dieses optional ein Septum auf.

Die Probenkammer, speziell eine der Kammerwände und insbesondere gerade nicht diejenige Kammerwand, welche von der Untersuchungseinheit angefahren und verformt wird, weist vorteilhafterweise eine oder mehrere Öffnungen auf, welche an den Medienanschluss angeschlossen sind, um mit diesem das Medium in den Innenraum zu führen und/oder aus dem Innenraum abzuführen. Vorzugsweise sind zwei Öffnungen vorhanden, eine für den Vorlauf und eine für den Rücklauf, um den Innenraum mit dem Medium zu durchströmen und die Probe gleichsam zu umspülen. Die Öffnungen sind jeweils vorteilhafterweise lediglich als einfache, insbesondere kreisrunde Löcher ausgeführt, welche gegenüber dem Medienanschluss (speziell Vor- und Rücklauf) ein Untermaß aufweisen, d.h. insbesondere einen geringeren Durchmesser, sodass eine besonders dichte Presspassung realisiert ist und die Probenkammer optimal abgedichtet ist. Der Medienanschluss ist beispielsweise als ein oder mehrere Nippel ausgebildet, über welche die Öffnungen der Probenkammern gestülpt werden. Durch das Untermaß ist das Funktionsprinzip einer radialen O-Ring-Dichtung imitiert. Somit ist die Probenkammer an das Fluidiksystem angeschlossen und dadurch zu jedem Zeitpunkt konstant und leckfrei mit Medium versorgbar.

Zweckmäßigerweise weist die Probenkammer eine Anlagekontur auf, insbesondere einen Kragen, Flansch oder Ähnliches, welche seitlich der Probenkammer absteht und gegenüber den Kammerwänden vorsteht. Die Anlagekontur ist vorzugsweise an einer Oberseite der Probenkammer ausgebildet. Die Anlagekontur umläuft die Probenkammer nicht notwendigerweise vollständig, zweckmäßigerweise aber überwiegend und bei einer Quaderform insbesondere auf drei von vier Seiten. Ist die Probenkammer in die Halterung eingesetzt (eingesetzter Zustand), dann liegt die Anlagekontur auf einem entsprechend ausgebildeten Vorsprung der Halterung auf. Dieser Vorsprung ist insbesondere analog zur Anlagekontur ausgebildet, z.B. als einfache Auflagefläche, welche die Probenkammer umläuft. Somit liegt die Probenkammer in einem festen Rahmen (nämlich dem Vorsprung) in der Halterung. Die Anlagekontur der Probenkammer verhindert in Verbindung mit dem Vorsprung der Halterung ein Durchrutschen der Probenkammer nach unten, da die Anlagekontur in dieser Richtung auf dem Vorsprung aufliegt. In dieser Richtung kann somit ein Anpressdruck zum dichten Verschließen der Probenkammer ausgeübt werden.

In einer vorteilhaften Ausgestaltung ist die Probenkammer mehrteilig ausgebildet und hierzu aus zwei Gehäuseteilen zusammengesetzt, insbesondere aus einem Unterteil und einem Deckel. Beispielsweise ist einer der Gehäuseteile ein kastenförmiges Unterteil, z.B. aus fünf von sechs Kammerwänden gebildet, und das andere Gehäuseteil ist ein Deckel, welcher dann die sechste Kammerwand bildet. Der Deckel oder alternativ oder zusätzlich eine oder mehrere der anderen Kammerwände sind zur Ausbildung als elastische Kammerwand wie oben beschrieben geeignet. Die mehrteilige Ausgestaltung der Probenkammer ermöglicht ein einfaches Einsetzen der Probe in den Innenraum sowie ein anschließendes Verschließen des Innenraums, indem die beiden Gehäuseteile miteinander verbunden werden, sodass der Innenraum abgeschlossen ist. Die oben genannte Anlagekontur ist insbesondere am Unterteil ausgebildet, insbesondere an einer Oberseite des Unterteils und somit nahe dem Deckel.

Vorzugsweise weist eines der Gehäuseteile eine umlaufende Dichtung auf, welche zum Abdichten insbesondere des Innenraums an dem anderen Gehäuseteil anliegt. Die Dichtung ist insbesondere einstückig, d.h. monolithisch mit dem einen Gehäuseteil hergestellt und somit auch aus dem gleichen Material. Alternativ ist die Dichtung ein separates Bauteil. Die Dichtung ist z.B. eine umlaufende Erhebung mit z.B. halbrundem Querschnitt entlang einer Oberkante des Gehäuseteils. Diese Oberkante wird vorzugsweise durch die oben beschriebene Anlagekontur gebildet, sodass die Dichtung dann auf der Anlagekontur ausgebildet ist. Bei der Dichtung wird zweckmäßigerweise ebenfalls das Prinzip einer axial vorgespannten O-Ring-Dichtung genutzt. Das Gehäuseteil mit Dichtung und das andere Gehäuseteil werden zusammengesetzt, sodass die Dichtung am anderen Gehäuseteil anliegt und die beiden Gehäuseteile somit dicht aneinander anliegen. Insbesondere sind in eingesetzten Zustand die Anlagekontur und die Dichtung zwischen dem Rahmen des Deckels und dem Vorsprung der Halterung eingespannt. Auf diese Weise ist sichergestellt, dass die nötige Vorspannung auf die Dichtung aufgebracht wird, ohne dass sich die Probenkammer dabei einfach nach unten (in vertikaler Richtung) verformt. Dadurch ist die Probenkammer vollständig geschlossen und kann für die Kultivierung von Proben und die multimodale Echtzeitbildgebung genutzt werden.

Speziell in Kombination mit der Dichtung ist es vorteilhaft, wenn die Fixierelemente ein Untermaß aufweisen, um eine definierte Vorspannung auf die Dichtung und zwischen den beiden Gehäuseteilen zu erzeugen. Unter "Untermaß" wird hierbei insbesondere verstanden, dass ein Abstand vom Grund der Halterung bis zu den Haltefortsätzen der Fixierelemente geringer ist als eine Höhe der Probenkammer in einem spannungslosen, unkomprimierten Zustand. Insbesondere wird dann beim Einsetzen in die Halterung die Dichtung zusammengedrückt und komprimiert, wodurch eine rückstellende Federkraft erzeugt wird, welche die Probenkammer in der Halterung verklemmt.

In die Probenkammer, insbesondere in deren Wandung, ist vorteilhafterweise eine Elektrodenanordnung integriert, zur Beaufschlagung der Probe mit einem elektrischen Feld. Da die Reifung einer Vielzahl von biologischen Geweben zusätzlich zu optimalen Kulturbedingungen (z.B. Temperatur und Nährstoffversorgung) gegebenenfalls auch elektrische, magnetische oder elektromagnetische Stimuli benötigt, ist eine Probenkammer mit Elektrodenanordnung vorteilhaft. Die Elektrodenanordnung ist entweder vollständig vom Innenraum und somit von einem darin gegebenenfalls angeordneten Medium isoliert (zur Erzeugung von stromlosen Feldern), oder steht in Kontakt mit dem Medium (zur Erzeugung von ionischen Strömen).

Während der Kultivierung und/oder der Untersuchung der Probe ist diese dann entsprechend mit einem elektrischen und/oder magnetischen Feld beaufschlagbar und wird dadurch stimuliert. Geeigneterweise ist die Elektrodenanordnung aus einer oder mehreren Elektroden gebildet, welche vorzugsweise jeweils aus leitfähigem Silikon hergestellt sind. Speziell bei einer Herstellung der Probenkammer aus Polydimethylsiloxan oder LSR lässt sich die Elektrodenanordnung dann stoffschlüssig und insbesondere einstückig, d.h. monolithisch, in die Wandung integrieren. Beispielsweise sind zwei Elektroden in zwei gegenüberliegende Kammerwände integriert, sodass durch den gesamten Innenraum hindurch von der einen zur anderen Kammerwand ein elektrisches Feld erzeugbar ist. Bei der Herstellung wird die Elektrodenanordnung dann insbesondere mit dem Material für die Wandung angegossen und dadurch in die Wandung integriert. Zum elektrischen Anschließen werden dann entsprechende elektrische Kontakte, z.B. Kontaktnadeln, einfach in die Wandung eingestochen, um mit der Elektrodenanordnung in Kontakt zu treten. Dabei dringen die elektrischen Kontakte aber insbesondere nicht bis in den Innenraum vor. Die elektrischen Kontakte sind z.B. in die Halterung des Bioreaktorsystems integriert, vorzugsweise derart, dass die Elektrodenanordnung automatisch beim Einsetzen der Probenkammer in die Halterung angeschlossen wird.

Nach der Untersuchung und allgemein am Ende der Verwendung der Probenkammer, kann diese wieder aus der Halterung entnommen und entsorgt werden (single-use-System).

Das hier beschriebene Bioreaktorsystem eignet sich auch zur Parallelisierung, d.h. zur parallelen Verarbeitung mehrerer Probenkammern mit je einer Probe. Hierfür weist das Bioreaktorsystem in einer geeigneten Ausgestaltung mehrere Halterungen wie beschrieben auf, jeweils für eine Probenkammer mit einer Probe. Grundsätzlich ist es möglich, für jede Probenkammer auch eine eigene Untersuchungseinheit vorzusehen, zweckmäßiger ist aber eine Ausgestaltung, bei welcher mit derselben Untersuchungseinheit mehrere Probenkammern angefahren werden. Insbesondere ist die Untersuchungseinheit dann derart entlang eines zweiten Verfahrwegs verfahrbar, dass die Proben nacheinander untersuchbar sind. Es erfolgt demnach eine sequentielle Untersuchung der Proben. Der erste und der zweite Verfahrweg sind beispielsweise senkrecht zueinander.

Die Verfahrbarkeit der Untersuchungseinheit ist relativ zur Probenkammer zu verstehen, d.h. die Untersuchungseinheit ist feststehend und die Probenkammer (oder die mehreren Probenkammern) werden bewegt oder umgekehrt ist die Probenkammer (sind die mehreren Probenkammern) feststehend und die Untersuchungseinheit wird bewegt. Die Verfahrbarkeit ist beispielsweise mittels eines geeigneten Linearantriebs realisiert.

Bei einer erfindungsgemäßen Verwendung wird eine Probenkammer wie vorstehend beschrieben zur umbettungsfreien Präparierung, Kultivierung und Untersuchung einer Probe verwendet insbesondere unter Beibehaltung von Bezugskoordinaten der Probe relativ zur Probenkammer. Die Probe wird zunächst in der Probenkammer präpariert, anschließend wird die Probenkammer insbesondere geschlossen. Ab diesem Punkt ist kein Eingriff in den Innenraum mehr erforderlich, sodass eine Kontamination der Probe im Weiteren vermieden wird. Die Probe wird nun in derselben Probenkammer zuerst kultiviert und schließlich untersucht. Bereits während der Kultivierung oder sogar schon während der Präparierung ist die Probenkammer in die Halterung des Bioreaktorsystems eingesetzt. Insbesondere bei der Präparierung der Probe wird deren räumliche Positionierung relativ zur Probenkammer festgelegt, sodass sich bestimmte Bezugskoordinaten (z.B. Abstände zu den Kammerwänden) der Probe relativ zur Probenkammer ergeben. Diese Bezugskoordinaten bleiben während der Präparierung, der Kultivierung und der Untersuchung vorteilhaft erhalten, d.h. unverändert. Die Kultivierung erfolgt mit dem Bioreaktorsystem, insbesondere über den Medienanschluss und/oder über einen oder mehrere andere Aktoren. Bereits während der Kultivierung kann die Probe schon untersucht werden, insbesondere wird dadurch die Kultivierung der Probe überwacht. Nach der Kultivierung wird die Probe dann mit der Untersuchungseinrichtung untersucht, z.B. wird eine Reifungsanalyse durchgeführt. Gegebenenfalls wird die Probe sogar zu mehreren verschiedenen Zeitpunkten untersucht, um eine zeitliche Entwicklung der Probe zu untersuchen. Während der Präparierung, Kultivierung und/oder der Untersuchung wird die Probe optional auch stimuliert, z.B. mittels der bereits beschriebenen Elektrodenanordnung.

Die genannte Verwendung der Probenkammer ist analog auch anwendbar auf das Bioreaktorsystem insgesamt, welches dann entsprechend zur umbettungsfreien Präparierung, Kultivierung und Untersuchung einer Probe oder sogar mehrerer Proben parallel verwendet wird.

Typischerweise sind in den Bereichen Biofabrikation und dreidimensionale Gewebekultur die Probenvorbereitung (z.B. 3D-Druck eines Biofabrikats, additive Herstellung), die Kultivierung und die anschließende optische Reifungsanalyse stets räumlich getrennt. Der Prozess beginnt mit der Überführung des Gewebekonstrukts in ein z.B. aus Polystyrol bestehendes, Kultivierungsgefäß. Dieses wird anschließend in einen Zellinkubator eingebracht und nach einer spezifischen Kultivierungsperiode von dem Inkubator zur optischen Analyse (i.d.R. räumlich getrennt) transferiert. Dies hat zur Folge, dass die optische Reifungsanalyse des Fabrikats nur einen einzelnen Zeitpunkt des Reifungsprozesses darstellt und der Transfer von Inkubator zur Mikroskopie kurzzeitig die Kultivierungsbedingungen mit nicht vorhersehbaren Konsequenzen unterbricht. Die hier vorgestellte Probenkammer und das ebenfalls hier vorgestellte Bioreaktorsystem insgesamt ermöglichen nun, alle diese Verfahrensschritte in einem gemeinsamen Verfahren zu kombinieren. Sobald sich die Probe in der Probenkammer befindet (Biofabrikate aus dem 3D-Drucker können auch direkt in die Probenkammer gedruckt werden), kann die Probenkammer geschlossen werden und die Kultivierung gestartet werden. Der Transport von der Präparierungseinheit (z.B. 3D-Drucker) hin zur Untersuchungsumgebung z.B. Mikroskopie-Umgebung sowie die optische Reifungsanalyse selbst erfolgen somit unter kontinuierlicher Kultivierung bei optimalen Bedingungen.

Die Aufgabe wird insbesondere auch gelöst durch ein Verfahren zum Betrieb eines Bioreaktorsystems wie oben beschrieben. Vorteilhafte Ausgestaltungen für das Verfahren ergeben sich analog aus dem oben Gesagten, speziell ergeben sich bevorzugte Verfahrensschritte analog zu der oben beschriebenen Verwendung sowie aus den oben insgesamt implizit oder explizit angegebenen Verfahrensschritten.

Die Erfindung betrifft insbesondere auch ein Herstellungsverfahren für eine Probenkammer wie oben beschrieben, die bisherigen Ausführungen gelten insofern analog auch für das Herstellungsverfahren. Bei dem Herstellungsverfahren wird im Kern wenigstens eine Kammerwand der Probenkammer aus einem elastischen Material hergestellt, sodass diese Kammerwand dann elastisch ist.

Die Erfindung findet insbesondere Verwendung im Bereich der regenerativen Medizin, der dreidimensionalen Zellkultur und der hiermit verbundenen Analyse von multizellulären Konstrukten und Geweben. Vorteilhaft ist vor allem auch die Einweg-Charakteristik der Probenkammer.

Zusammenfassend wurde ein Bioreaktorsystem mit flexibler, transparenter Probenkammer zur optischen 4D-Bioanalytik von z.B. Gewebekonstrukten vorgestellt. Die Probenkammer ist insbesondere zur Einbringung in eine miniaturisierte Bioreaktorumgebung geeignet, d.h. das Bioreaktorsystem ist dann als Mikrobioreaktorsystem ausgebildet. Die Probenkammer ermöglicht die optische Analyse dreidimensionaler Proben, z.B. Gewebekonstrukte, über die Zeit (vierte Dimension), insbesondere unter dem Aspekt der reproduzierbar exakt wieder anfahrbaren kartesischen Koordinaten (insbesondere die Bezugskoordinaten) der Probe. Die Probenkammer ermöglicht weiter auch die elektrische, magnetische und/oder elektromagnetische Stimulation einer Probe durch elastische, d.h. flexible Kammerwände (auch: Fenster) und mittels Elektrodenanordnung (insbesondere Polymerelektroden). Mit anderen Worten: die Erfindung ermöglicht vorteilhaft die Stimulation und insgesamt vierdimensionale Analyse von Gewebeproben, z.B. während Kultivierung, Gewebereifung, Alterungsprozessen usw., insbesondere in einer Miniatur-Bioreaktorumgebung. Durch die Fertigung der Probenkammer aus transparentem Silikon (LSR) wird ein optischer Tiefen-Zugang zur kultivierten Probe gewährleistet. Zugleich ermöglicht die Elastizität des wenigstens für die elastische Kammerwand gewählten Materials ebenfalls eine axiale Fokussierung durch das gesamte Volumen der Probe. Ein (Mikroskop-)Objektiv kann somit direkt mit der Kammerwand in Kontakt gebracht und durch deren elastischen Verformung tiefer in die Probenkammer hinein fokussieren. Ferner kann die Wandung der Probenkammer im Allgemeinen zusätzlich noch durch die Einbringung von z.B. Polymerelektroden in eine oder mehrere der Kammerwände funktionalisiert werden, um die Probe elektrisch, magnetisch oder elektromagnetisch zu stimulieren. Die Baugröße der Probenkammer eröffnet zusätzlich die Möglichkeit, Gewebe direkt in den Innenraum zu drucken, additiv zu fertigen oder sonstig zu applizieren und ohne zusätzliche Zwischenschritte direkt in die Kultivierung zu überführen. Insgesamt ist somit insbesondere ein Bioreaktorsystem mit modularer, flexibler und optisch zugänglicher Probenkammer realisiert, welche eine axiale Fokussierung bis in tiefe Gewebeschichten der Probe ermöglicht und optional auch eine z.B. elektromagnetische Stimulation zu jedem beliebigen Zeitpunkt, auch bereits vor der Untersuchung der Probe.

Ferner ist die Probenkammer geeignet, in eingesetztem Zustand, d.h. in der Halterung einsitzend, direkt in eine Präparationsvorrichtung, z.B. einen Bioprinter eingebracht zu werden. Das gesamte Bioreaktorsystem mit Probenkammer wird also zur Präparierung einer Probe innerhalb der Probenkammer in die Präparationsvorrichtung eingeführt. Dadurch ist ein nahtloser Übergang von Präparierung (z.B. Bioprinting) zu Kultivierung und anschließender und/oder simultaner, z.B. bildgebender Reifungsanalyse realisiert. Während der Präparierung ist die Probenkammer bereits in die Halterung des Bioreaktorsystems eingesetzt. Damit wird eine feste Koordinatenbeziehung von Probe und Analytik ermöglicht, d.h. eine Beibehaltung von Bezugskoordinaten der Probe relativ zur Probenkammer, welche in mehrstufigen und räumlich getrennten Prozessen i.d.R. verloren geht.

Nachfolgend werden Ausführungsbeispiele der Erfindung anhand einer Zeichnung näher erläutert. Darin zeigen jeweils schematisch:
- Fig. 1: ein Bioreaktorsystem,
- Fig. 2: eine Probenkammer,
- Fig. 3: die Probenkammer aus Fig. 2 in einer anderen Ansicht,
- Fig. 4: die Probenkammer aus Fig. 2 in einer anderen Ansicht,
- Fig. 5: die Probenkammer aus Fig. 2 in einer anderen Ansicht,
- Fig. 6: einen Ausschnitt eines Bioreaktorsystems in einer Schnittansicht,
- Fig. 7: einen Ausschnitt eines Bioreaktorsystems in einer perspektivischen Ansicht,
- Fig. 8: eine alternative Probenkammer,
- Fig. 9: die Probenkammer aus Fig. 8 in einer anderen Ansicht,
- Fig. 10: die Präparierung einer Probe in einer Probenkammer,
- Fig. 11: die Kultivierung der Probe in der Probenkammer aus Fig. 10,
- Fig. 12: die Untersuchung der Probe in der Probenkammer aus Fig. 11 (hier beispielhaft mit Lichtblatt-Mikroskopie).

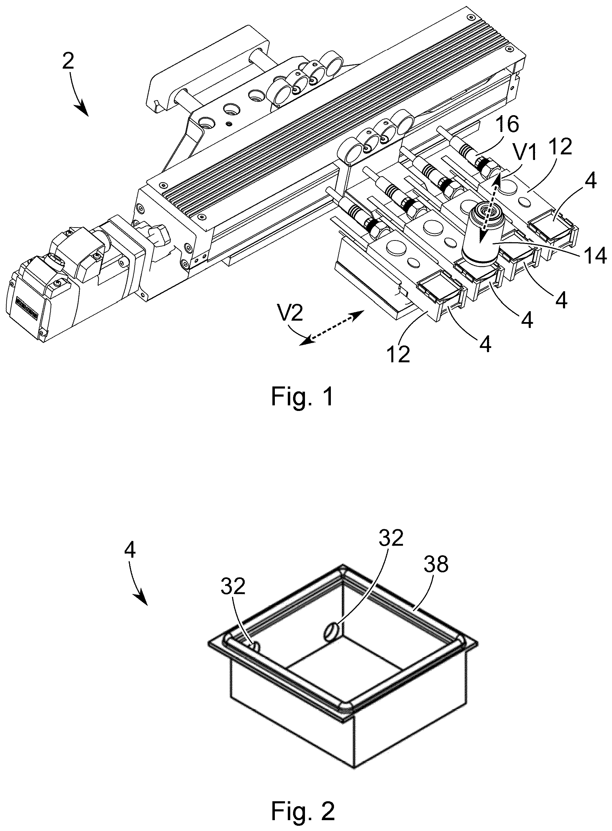

Ein Ausführungsbeispiel für ein erfindungsgemäßes Bioreaktorsystem 2 ist in Fig. 1 in einer perspektivischen Ansicht gezeigt. Das Bioreaktorsystem 2 weist hier beispielhaft vier Probenkammern 4 auf, jeweils mit einem Innenraum 6 zum Aufnehmen einer Probe 8. Die Probenkammer 4 weist hierfür eine Wandung 10 auf, welche den Innenraum 6 umschließt und dessen Form definiert. Die Probe 8 ist z.B. ein Biofabrikat und/oder Gewebekonstrukt. Das Bioreaktorsystem 2 weist zudem hier beispielhaft auch vier Halterungen 12 auf, in welche je eine der Probenkammern 4 formschlüssig und dicht eingesetzt ist. Im eingesetzten Zustand ist die Probenkammer 4 in der Halterung 12 fixiert. Schließlich weist das Bioreaktorsystem 2 noch eine Untersuchungseinheit 14 auf, zur Untersuchung der Probe 8, während diese in der Probenkammer 4 aufgenommen ist. Mit der Untersuchungseinheit 14 wird die Probe 8 im Betrieb untersucht. Die Untersuchungseinheit 14 ist ein Teil einer nicht weiter dargestellten Messvorrichtung des Bioreaktorsystems 2, mit welchem die Probe 8 analysiert wird. Die Untersuchungseinheit 14 ist vorliegend außerhalb der Probenkammer 4 angeordnet.

In eine jeweilige Halterung 14 sind nach Bedarf noch ein oder mehrere Sensoren und/oder Aktoren integriert, um je nach Bedarf die Probe 8 und/oder deren Umgebung (z.B. ein Medium im Innenraum 6) zu untersuchen, zu stimulieren und/oder zu manipulieren. In Fig. 1 ist beispielhaft ein Temperatursensor 16 zur Messung der Temperatur der Probenkammer 4 gezeigt.

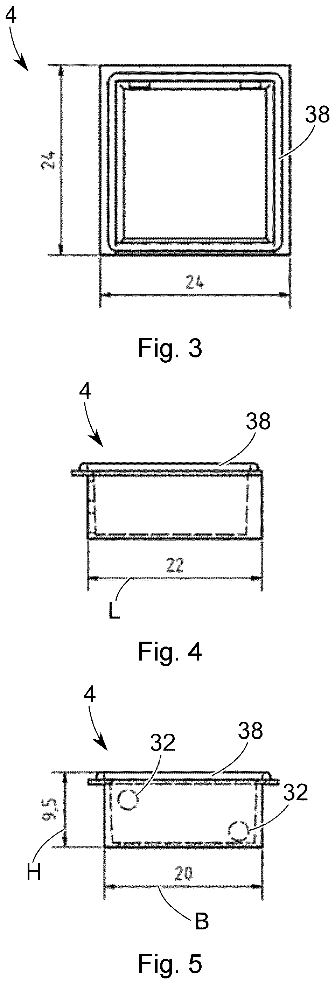

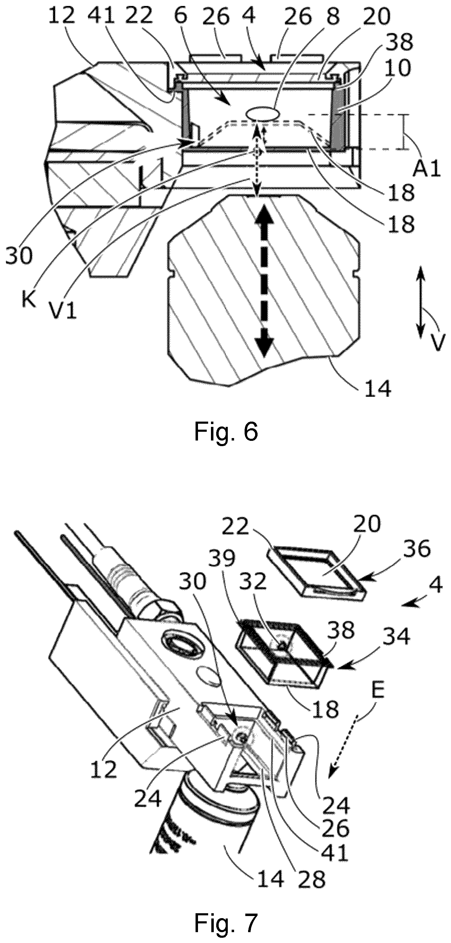

Ein Ausführungsbeispiel für die Probenkammer 4 ist im Detail (jedoch ohne Deckel) in den Fig. 2, 3, 4 und 5 in unterschiedlichen Ansichten gezeigt, nämlich in Fig. 2 in einer perspektivischen Ansicht, in Fig. 3 in einer Draufsicht und in den Fig. 4 und 5 je in einer Seitenansicht senkrecht zueinander und senkrecht zur Draufsicht. Die Probenkammer 4 weist wenigstens eine (erste) Kammerwand 18 auf, welche ganz oder teilweise elastisch ist, um beim Verfahren der Untersuchungseinheit 14 entlang eines ersten Verfahrwegs V1 durch die Untersuchungseinheit 14 verformt zu werden. Dies ist beispielhaft in Fig. 6 illustriert, welche die Probenkammer 4 in der Halterung 12 in einer Seitenansicht zeigt. In Fig. 6 ist die Verformung der Kammerwand 18 beim Anfahren mit der Untersuchungseinheit 14 als Strichlinie gezeigt. In Fig. 6 wird die Probenkammer 4 von unten angefahren, hingegen ist in Fig. 1 die Untersuchungseinheit 14 auf der gegenüberliegenden Seite gezeigt, d.h. die Probenkammer wird von oben angefahren. Jegliche Seiten, speziell solche, welche der Reaktorrückwand abgewandt sind, sind grundsätzlich möglich.

Die erste Kammerwand 18 wird auch als "elastische Kammerwand" bezeichnet. Die Kammerwand 18 ist ein Teil der Wandung 10 der Probenkammer 4. In einem unverformten Zustand ist die Kammerwand 18 wie in Fig. 6 erkennbar plan, d.h. flach oder eben. Deutlich wird auch, dass die Probenkammer 4 mehrere Kammerwände 18, 20 aufweist, welche zusammen die Wandung 10 bilden und den Innenraum umschließen. Mehrere oder sämtliche Kammerwände 18, 20 der Probenkammern können grundsätzlich gleichartig ausgebildet sein. Die hier beispielhaft gezeigte Probenkammer 4 ist insgesamt quaderförmig, mit dann sechs Kammerwänden 18, 20, von welchen dann wenigstens eine elastisch ist. Andere Formen für die Probenkammer 4 und andere Anzahlen an Kammerwänden 18, 20 sind aber ebenso möglich.

Aufgrund der elastischen Kammerwand 18 ist die Untersuchungseinheit 14 deutlich näher an die Probe 8 heranfahrbar und auch ein größerer Verfahrweg V1 ist möglich als im Vergleich zu einer nicht-elastischen und somit nicht-verformbaren Kammerwand. Im hier gezeigten Ausführungsbeispiel ist die Untersuchungseinheit 14 ein Objektiv, speziell ein Mikroskop-Objektiv, für eine optische Untersuchung der Probe 8. Die Probe 8 ist innerhalb der Probenkammer 4 nicht zwingend unmittelbar an der elastischen Kammerwand 18 positioniert, sondern in einem Abstand A1 davon, z.B. wie in Fig. 6 angedeutet im Zentrum der Probenkammer 4 oder alternativ an einer der elastischen Kammerwand 18 gegenüberliegenden Kammerwand 20. Dieser Abstand A1 wird nun durch das Anfahren der elastischen Kammerwand 18 und deren Verformung durch die Untersuchungseinheit 14 reduziert.

Die Wandung 10 der Probenkammer 4 ist vorliegend dünnwandig, mit einer Dicke im Bereich von 0,2 mm bis 1 mm. Die Probenkammer 4, genauer deren Innenraum 6, weist vorliegend beispielhaft ein Volumen von etwa 3 - 4 ml auf, bei Abmessungen (Höhe H, Breite B, Länge L) der Probenkammer 4 im Bereich von wenigen Millimetern. Die gezeigten Abmessungen bis in den zweistelligen Millimeterbereich (≥ 10 mm) hinein, stellen eine besondere Herausforderung für die optische Untersuchung dar, da hochauflösende Objektive in der Regel über Arbeitsabstände im einstelligen Millimeterbereich (< 10 mm) verfügen. Die hier gezeigte Untersuchungseinheit 14 weist einen Arbeitsabstand auf, welcher geringer als der Abstand A1 zwischen der Kammerwand 18 in unverformtem Zustand und einer Probenposition für die Probe 8 innerhalb der Probenkammer 4 ist. Um diese Herausforderung zu umgehen, sind vorliegend eine oder mehrere der Kammerwände 18, 20 elastisch, d.h. aus einem elastischen Material gefertigt, welches eine elastische Verformung durch das Objektiv erlaubt. In unverformtem Zustand der Probenkammer 4 liegt die elastische Kammerwand 18 entlang des ersten Verfahrwegs V1. Beim Anfahren der elastischen Kammerwand 18 mit der Untersuchungseinheit 14 stößt diese an die Kammerwand 18 an und drückt entlang des Verfahrwegs V1 wie in Fig. 6 erkennbar auf die Kammerwand 18, sodass diese entsprechend verformt wird. Der Verfahrweg V1 weist demnach einen Kontaktpunkt K auf, welcher den Verfahrweg V1 in zwei aufeinanderfolgende Abschnitte teilt, nämlich einen ersten Abschnitt, entlang welchem die Untersuchungseinheit 14 ohne jeglichen Kontakt zur Kammerwand 18 verfahren wird, und einen zweiten Abschnitt, entlang welchem die Untersuchungseinheit 14 an der Kammerwand 18 anliegt und diese beim weiteren Verfahren entsprechend verformt. Dabei liegt die Untersuchungseinheit 14 an der Kammerwand 18 auf einer Kontaktfläche an, welche geringer ist als eine Gesamtfläche der Kammerwand 18.

Wie in Fig. 6 erkennbar ist, wird die Kammerwand 18 beim Anfahren in den Innenraum 6 hinein und auf die Probe 8 zu verformt und der erste Verfahrweg V1 verläuft senkrecht zur Kammerwand 18. Die elastische Kammerwand 18 ist auch reversibel verformbar, d.h. beim Zurückfahren der Untersuchungseinheit 14 entlang des Verfahrwegs V1 nimmt die Kammerwand 18 einen ursprünglichen, unverformten Zustand wieder an. Die elastische Kammerwand 18 ist auch zerstörungsfrei verformbar und wird demnach nicht durch die Untersuchungseinheit 14 beschädigt, sondern bleibt intakt und geschlossen, sodass auch der Innenraum 6 der Probenkammer 4 insgesamt geschlossen bleibt.

Im gezeigten Ausführungsbeispiel sind alle Kammerwände 18, 20 der Probenkammer 4 als elastische Kammerwand ausgebildet, mit dem Bezugszeichen 18 wird dann speziell diejenige elastische Kammerwand bezeichnet, welche auch tatsächlich in der jeweiligen Figur von der Untersuchungseinheit 14 angefahren und verformt wird. Alle übrigen Kammerwände 20 sind aber ebenso elastisch, sodass für ein Medium im Innenraum 6 eine entsprechende Ausweichmöglichkeit vorhanden ist.

Die elastische Kammerwand 18 (hier sogar alle Kammerwände 18, 20) ist vorliegend dadurch elastisch, dass diese aus einem elastischen Material hergestellt ist, speziell aus Polydimethylsiloxan oder Liquid Silicone Rubber (kurz: LSR, ein flexibles Silikon). Die elastische Kammerwand 18 ist auch lichtdurchlässig und transparent, sodass die Probe 8 durch die Kammerwand 18 hindurch optisch untersucht werden kann. Die jeweils winklig zur Kammerwand 18 angeordneten Kammerwände 20 der Probenkammer 4 sind ebenfalls lichtdurchlässig, zur optischen Untersuchung der Probe 8 z.B. mittels Lichtblattmikroskopie. Bei der optischen Untersuchung wird die Probe 8 dann durch diese weitere Kammerwand 20 seitlich mit einem Lichtblatt beleuchtet und insbesondere senkrecht dazu beobachtet.

Im gezeigten Ausführungsbeispiel bestehen jegliche Teile der Probenkammer 4, welche an den Innenraum 6 angrenzen aus demselben Material, nämlich PDMS oder LSR. Dieses Material ist elastisch sowie lichtdurchlässig und/oder transparent und biokompatibel. Andere Teile der Probenkammer 4, welche nicht an den Innenraum 6 angrenzen, sondern entsprechend außenseitig liegen, speziell die unten noch näher beschriebene Haltekontur 22, können hingegen aus einem anderen Material bestehen.

Vorliegend ist die Probenkammer 4 austauschbar, d.h. lösbar in der Halterung 12 fixiert und bei Bedarf aus der Halterung 12 entnehmbar, um dann eine andere Probenammer 4 in diese Halterung 12 einzusetzen und entsprechend eine andere Probe 8 zu untersuchen. Verdeutlicht wird dies in Fig. 7, welche die hier zweiteilige Probenkammer 4 abseits der Halterung 12 zeigt, zum Einsetzen in diese entlang einer Einsetzrichtung E. Auch in Fig. 7 erfolgt die Untersuchung mittels der Untersuchungseinheit 14 wie in Fig. 6 auch von unten.

Die hier gezeigte Probenkammer 4 weist eine Haltekontur 22 auf, vorliegend einen Rahmen, aus einem starren Material auf, zur (lösbaren) Fixierung der Probenkammer 4 an hier vier Fixierelementen 24 der Halterung 12. Die Haltekontur 22 und die Fixierelemente 24 bilden einen Fixiermechanismus, hier einen Rastmechanismus. Die Fixierelemente 24 weisen jeweils einen Haltefortsatz 26 auf und sind hierzu vorliegend als Schnapphaken oder als Rasthaken ausgebildet. Die Haltefortsätze 26 hintergreifen die Haltekontur 22 (vgl. Fig. 6) und fixieren auf diese Weise die Probenkammer 4 in der Halterung 12 und halten die Probenkammer 4 in einer bestimmten Position fest. Die Probenkammer 4 wird dann in einer Einsetzrichtung E in die Halterung 12 eingesetzt, dabei drückt die Haltekontur 22 die Haltefortsätze 26 der Fixierelemente 24 zur Seite und gleitet an diesen vorbei. Sobald die Haltekontur 22 die Haltefortsätze 26 passiert hat, rasten die Fixierelemente 24 an der Haltekontur 22 ein und fixieren die Probenkammer 4 in Verbindung mit einem Grund 28 der Halterung 12, welcher als Gegenkontur zur Fixierung dient. Durch entsprechendes Aufbiegen der Fixierelemente 24 wird die Probenkammer 4 dann wieder gelöst und kann entgegen der Einsetzrichtung E wieder entnommen werden.

Die Halterung 12 weist vorliegend auch einen Medienanschluss 30 auf, zur Zufuhr und/oder Abfuhr eines Mediums. Der Medienanschluss 30 weist einen Vorlauf und einen Rücklauf auf, hier in Form von je einem Nippel. Die Probenkammer 4 weist dann entsprechend zwei Öffnungen 32 auf, welche an den Medienanschluss 30 angeschlossen sind, um mit diesem das Medium in den Innenraum 6 zu führen und/oder daraus abzuführen. Die Öffnungen 32 sind jeweils lediglich als einfache, kreisrunde Löcher ausgeführt, welche gegenüber Vor- und Rücklauf ein Untermaß aufweisen.

Die hier gezeigte Probenkammer 4 ist mehrteilig ausgebildet und aus zwei Gehäuseteilen 34, 36 zusammengesetzt, nämlich aus einem Unterteil 34 und einem Deckel 36, welche beide explizit in Fig. 7 erkennbar sind. Das Unterteil 34 ist kastenförmig und aus fünf von sechs Kammerwänden 18, 20 gebildet, und der Deckel 36 bildet die sechste Kammerwand 18, 20. In den Fig. 2 bis 5 ist nur das Unterteil 34 gezeigt. Eines der Gehäuseteile 34, 36, hier beispielhaft das Unterteil 34, weist zudem noch eine umlaufende Dichtung 38 auf, welche zum Abdichten des Innenraums 6 an dem anderen Gehäuseteil 36 anliegt. Die Dichtung 38 ist hier sogar einstückig, d.h. monolithisch mit dem Unterteil 34 hergestellt und somit auch aus dem gleichen Material. Die Dichtung 38 ist vorliegend beispielhaft eine umlaufende Erhebung mit halbrundem Querschnitt entlang einer Oberkante des Unterteils 34.

Bei dem hier gezeigten Ausführungsbeispiel weist die Probenkammer 4 eine Anlagekontur 39 auf, welche hier ein Kragen ist und seitlich (d.h. überwiegend senkrecht zur vertikalen Richtung V) der Probenkammer 4 absteht und gegenüber den Kammerwänden 18, 20 vorsteht. Die Anlagekontur 39 ist vorliegend an einer Oberseite der Probenkammer 4 ausgebildet und umläuft die Probenkammer 4 nicht vollständig, aber überwiegend und auf drei von vier Seiten. Ist die Probenkammer 4 z.B. wie in Fig. 6 in die Halterung 12 eingesetzt (eingesetzter Zustand), dann liegt die Anlagekontur 39 auf einem entsprechend ausgebildeten Vorsprung 41 der Halterung 12 auf. Dieser Vorsprung 41 ist analog zur Anlagekontur 39 ausgebildet, vorliegend als einfache Auflagefläche, welche die Probenkammer 4 umläuft. Somit liegt die Probenkammer 4 in einem festen Rahmen in der Halterung 12. Die Anlagekontur 39 der Probenkammer 4 verhindert somit in Verbindung mit dem Vorsprung 41 der Halterung 12 ein Durchrutschen der Probenkammer 4 nach unten, d.h. in vertikaler Richtung V, da die Anlagekontur 39 in dieser Richtung auf dem Vorsprung 41 aufliegt. In dieser Richtung kann somit ein Anpressdruck zum dichten Verschließen der Probenkammer 4 ausgeübt werden.

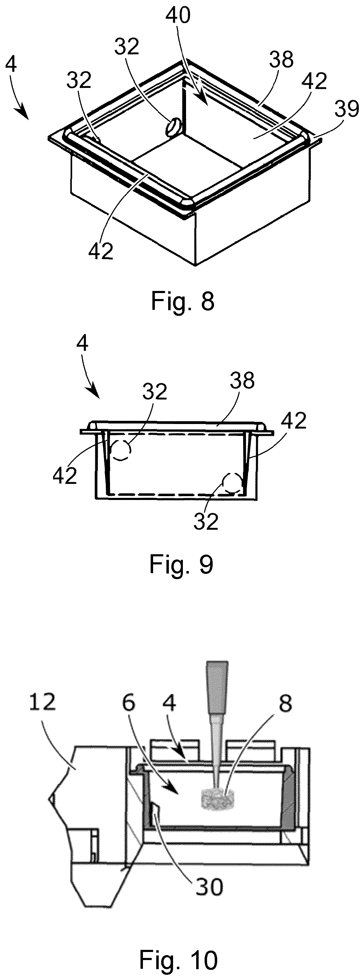

In die Probenkammer 4 ist optional eine Elektrodenanordnung 40 integriert, zur Beaufschlagung der Probe 8 mit einem elektrischen Feld. Ein Ausführungsbeispiel für eine solche Probenkammer 4 mit einer Elektrodenanordnung 40, welche beispielsweise zwei Elektroden 42 aufweist, ist in den Fig. 8 und 9 gezeigt, wobei Fig. 8 eine Ansicht wie in Fig. 2 zeigt und Fig. 9 eine Ansicht wie in Fig. 5. Der einzige Unterschied sind die zusätzlichen Elektroden 42, welche hier jeweils entlang einer gesamten Kammerwand 20 ausgebildet sind. Während der Kultivierung und/oder der Untersuchung der Probe 8 ist diese dann entsprechend mit einem elektrischen und/oder magnetischen Feld beaufschlagbar und wird dadurch stimuliert. Vorliegend ist die Elektrodenanordnung 40 aus mehreren Elektroden 42 gebildet, welche jeweils aus leitfähigem Silikon hergestellt sind und bei der Herstellung der Probenkammer 4 stoffschlüssig und einstückig in die Wandung 10 integriert sind.

Das hier beispielhaft gezeigte Bioreaktorsystem 2 eignet sich auch zur Parallelisierung, d.h. zur parallelen Verarbeitung mehrerer Probenkammern 4 mit je einer Probe 8, wie in Fig. 1 erkennbar ist. Grundsätzlich ist es möglich, für jede Probenkammer 4 auch eine eigene Untersuchungseinheit 14 vorzusehen, vorliegend werden aber mit derselben Untersuchungseinheit 14 mehrere Probenkammern 4 angefahren. Hierzu ist die Untersuchungseinheit 14 derart entlang eines zweiten Verfahrwegs V2 verfahrbar, dass die Proben 8 nacheinander untersuchbar sind.

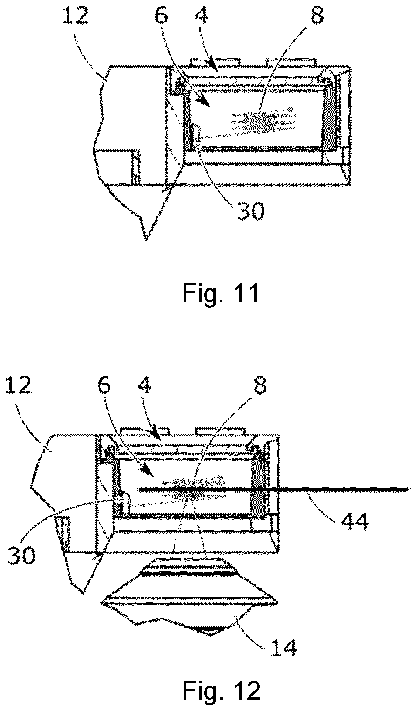

Anhand der Fig. 10, 11 und 12 wird nachfolgend ein Ausführungsbeispiel für eine erfindungsgemäße Verwendung der Probenkammer 4 zur umbettungsfreien Präparierung (Fig. 10), Kultivierung (Fig. 11) und Untersuchung (Fig. 12) einer Probe 8 beschrieben. Die Probe 8 wird zunächst wie in Fig. 10 gezeigt in der Probenkammer 4 präpariert, z.B. direkt in das Unterteil 34 hineingedruckt, anschließend wird die Probenkammer 4 geschlossen, indem der Deckel 36 aufgesetzt wird. Ab diesem Punkt ist kein Eingriff in den Innenraum 6 mehr erforderlich, sodass eine Kontamination der Probe 8 im Weiteren vermieden wird. Die Probe 8 wird nun in derselben Probenkammer 4 zuerst kultiviert, vgl. Fig. 11, welche auch mit einer Strichlinie angedeutet das Durchströmen des Innenraums 6 mit einem Medium zeigt. Schließlich wird die Probe 8 wie in Fig. 12 gezeigt untersucht, hier exemplarisch mittels Lichtblattmikroskopie. Dabei wird seitlich ein Lichtblatt 44 auf die Probe 8 gestrahlt und diese dann senkrecht zu dem Lichtblatt 44 mit der Untersuchungseinheit 14 untersucht. Während allen drei Schritten ist die Probenkammer 4 in die Halterung 12 des Bioreaktorsystems 4 eingesetzt. Die Kultivierung erfolgt mit dem Bioreaktorsystem 2 über den Medienanschluss 30. Bereits während der Kultivierung kann die Probe 8 auch schon untersucht werden.

### Bezugszeichenliste

- 2: Bioreaktorsystem
- 4: Probenkammer
- 6: Innenraum
- 8: Probe
- 10: Wandung
- 12: Halterung
- 14: Untersuchungseinheit
- 16: Temperatursensor
- 18: elastische Kammerwand
- 20: Kammerwand
- 22: Haltekontur
- 24: Fixierelement
- 26: Haltefortsatz
- 28: Grund
- 30: Medienanschluss
- 32: Öffnung
- 34: Unterteil (Gehäuseteil)
- 36: Deckel (Gehäuseteil)
- 38: Dichtung
- 39: Anlagekontur
- 40: Elektrodenanordnung
- 41: Vorsprung
- 42: Elektrode
- 44: Lichtblatt
- A1: Abstand (zwischen Probe und elastischer Kammerwand)
- B: Breite
- E: Einsetzrichtung
- H: Höhe
- K: Kontaktpunkt
- L: Länge
- V: vertikale Richtung
- V1: erster Verfahrweg
- V2: zweiter Verfahrweg

## Patentansprüche

1. Bioreaktorsystem (2),
a. welches eine Probenkammer (4) aufweist, mit einem Innenraum (6) zum Aufnehmen einer Probe (8),
b. welches eine Halterung (12) aufweist, in welche die Probenkammer (4) eingesetzt ist,
c. welches eine Untersuchungseinheit (14) aufweist, zur Untersuchung der Probe (8),
d. wobei die Probenkammer (4) wenigstens eine Kammerwand (18) aufweist, welche elastisch ist, um beim Verfahren der Untersuchungseinheit (14) entlang eines ersten Verfahrwegs (V1) durch die Untersuchungseinheit (14) verformt zu werden.

2. Bioreaktorsystem (2) nach Anspruch 1,
wobei die Untersuchungseinheit (14) außerhalb der Probenkammer (4) angeordnet ist,
wobei der erste Verfahrweg (V1) senkrecht zur Kammerwand (18) verläuft, wobei die Kammerwand (18) in unverformtem Zustand entlang des ersten Verfahrwegs (V1) liegt.

3. Bioreaktorsystem (2) nach Anspruch 1 oder 2,
wobei die Untersuchungseinheit (14) einen Arbeitsabstand aufweist, welcher geringer ist als ein Abstand (A1) zwischen der Kammerwand (18) in unverformtem Zustand und einer Probenposition für die Probe (8) innerhalb der Probenkammer (4).

4. Bioreaktorsystem (2) nach einem der Ansprüche 1 bis 3,
wobei wenigstens die Kammerwand (18) aus Liquid Silicone Rubber oder Polydimethylsiloxan hergestellt ist.

5. Bioreaktorsystem (2) nach einem der Ansprüche 1 bis 4,
wobei die Kammerwand (18) lichtdurchlässig und/oder transparent ist und wobei die Untersuchungseinheit (14) zur optischen Untersuchung der Probe (8) ausgebildet ist, insbesondere als Objektiv.

6. Bioreaktorsystem (2) nach einem der Ansprüche 1 bis 5,
wobei die Probenkammer (4) zusätzlich zu der elastischen Kammerwand (18) wenigstens eine weitere Kammerwand (20) aufweist, welche winklig, insbesondere senkrecht, zur elastischen Kammerwand (18) angeordnet ist und lichtdurchlässig ist, zur optischen Untersuchung der Probe (8).

7. Bioreaktorsystem (2) nach einem der Ansprüche 1 bis 6,
wobei die Probenkammer (4) austauschbar ist.

8. Bioreaktorsystem (2) nach einem der Ansprüche 1 bis 7,
wobei die Probenkammer (4) eine Haltekontur (22), insbesondere einen Rahmen, aus einem starren Material aufweist, zur Fixierung der Probenkammer (4) an einem oder mehreren Fixierelementen (24) der Halterung (12).

9. Bioreaktorsystem (2) nach einem der Ansprüche 1 bis 8,
wobei die Halterung (12) einen Medienanschluss (30) aufweist, zur Zufuhr und/oder Abfuhr eines Mediums,
wobei die Probenkammer (4) eine oder mehrere Öffnungen (32) aufweist, welche an den Medienanschlusses (30) angeschlossen sind, um mit diesem das Medium in den Innenraum (6) zu führen und/oder aus dem Innenraum (6) abzuführen.

10. Bioreaktorsystem (2) nach einem der Ansprüche 1 bis 9,
wobei jegliche Teile der Probenkammer (4), welche an den Innenraum (6) angrenzen, aus demselben Material bestehen, insbesondere aus einem elastischen, lichtdurchlässigen und biokompatiblen Material.

11. Bioreaktorsystem (2) nach einem der Ansprüche 1 bis 10,
wobei die Probenkammer (4) aus zwei Gehäuseteilen (34, 36) zusammengesetzt ist,
wobei eines der Gehäuseteile (34) eine umlaufende Dichtung (38) aufweist, welche zum Abdichten an dem anderen Gehäuseteil (36) anliegt.

12. Bioreaktorsystem (2) nach einem der Ansprüche 1 bis 11,
wobei in die Probenkammer (4) eine Elektrodenanordnung (40) integriert ist, zur Beaufschlagung der Probe (8) mit einem elektrischen und/oder magnetischen Feld.

13. Bioreaktorsystem (2) nach einem der Ansprüche 1 bis 12,
wobei dieses mehrere Halterungen (12) aufweist, jeweils für eine Probenkammer (4) mit einer Probe (8),
wobei die Untersuchungseinheit (14) derart entlang eines zweiten Verfahrwegs (V2) verfahrbar ist, dass die Proben (8) nacheinander untersuchbar sind.

14. Probenkammer (4) für ein Bioreaktorsystem (2) gemäß einem der Ansprüche 1 bis 13.

15. Verwendung einer Probenkammer (4) gemäß Anspruch 14 zur umbettungsfreien Präparierung, Kultivierung und Untersuchung einer Probe, insbesondere unter Beibehaltung von Bezugskoordinaten der Probe relativ zur Probenkammer.
